(19) Europäisches Patentamt
European Patent Office
Office européen des brevets

(11) Publication number: 0 299 728
A2

(12) # EUROPEAN PATENT APPLICATION

(21) Application number: 88306382.8

(22) Date of filing: 13.07.88

(51) Int. Cl.[4]: C07D 471/04 , //(C07D471/04, 221:00,205:00)

(30) Priority: 17.07.87 US 74667

(43) Date of publication of application: 18.01.89 Bulletin 89/03

(84) Designated Contracting States: AT BE CH DE ES FR GB GR IT LI NL SE

(71) Applicant: ELI LILLY AND COMPANY
Lilly Corporate Center
Indianapolis Indiana 46285(US)

(72) Inventor: Munroe, John Edwin
8001 Castle Cove Road
Indianapolis Indiana 46256(US)

(74) Representative: Tapping, Kenneth George et al
Erl Wood Manor
Windlesham Surrey, GU20 6PH(GB)

(54) **Carbonylation process for beta-lactam antibiotics.**

(57) 7β-Acylamino (or 7β-protected amino)-3-alkoxycarbonyl (or phenylalkoxycarbonyl)-1-carba(1-dethia)-3-cephem-4-carboxylic acid esters are provided via palladium catalyzed carbonylation in the presence of a $C_1$-$C_6$ alkanol or phenyl substituted $C_1$-$C_6$ alkanol of a 1-carba (1-dethia)-3-cephem-3-triflate ester.

EP 0 299 728 A2

## CARBONYLATION PROCESS FOR β-LACTAM ANTIBIOTICS

This invention relates to the preparation of β-lactam antibiotics. In particular, it relates to a process for preparing 7β-acylamino (or 7β-protected amino)-3-alkoxycarbonyl (or phenylalkoxycarbonyl)-1-carba(1-dethia)-3-cephem-4-carboxylic acids.

Among the newer β-lactam antibiotics currently under study are the 1-carba(1-dethia)-3-cephem-4-carboxylic acids. These β-lactam compounds are not amenable to ready synthesis, particularly in the isomeric form which possesses the most potent antibacterial activity. Numerous approaches to the elaboration of these ring systems have been investigated with varying degrees of success. Notable among the asymmetric routes is that described by Evans et al., U.S. Patent No, 4,665,171. Because of the importance of β-lactam antibiotics in the treatment of infectious disease, processes for the preparation of such antibiotics are likewise of much importance.

According to this invention there is provided a process for preparing a compound represented by the Formula 1

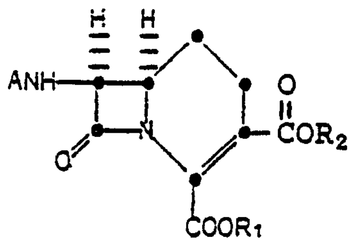

1

wherein A is an amino-protecting group or an acyl group R(CO)-; $R_1$ is a carboxy-protecting group; and $R_2$ is $C_1$-$C_6$ alkyl; or $C_1$-$C_6$ substituted by phenyl; which comprises reacting a 1-carba-3-triflate ester represented by the Formula 2

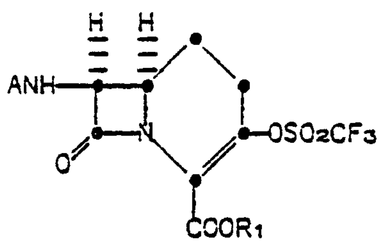

2

wherein A and $R_1$ are as defined above, in the presence of palladium (O) with carbon monoxide and an alcohol represented by the formula $R_2OH$, wherein $R_2$ is $C_1$-$C_6$ alkyl or $C_1$-$C_6$ alkyl substituted by phenyl.

The process can be carried out at a temperature between about -5° C and about 50° C, preferably at about 20° C to about 35° C.

Inert solvents which can be used are polar solvents of sufficient polarity to maintain substantially all of the reagents and reactants in solution. Polar solvents that can be used are dimethylformamide, dimethylacetamide, acetonitrile and like polar solvents.

The palladium catalyst employed in the process is soluble or partially soluble Pd(O) which can be generated in situ or provided in the form of a Pd(O) compound such as tetrakis-(triphenylphosphine)-palladium (O). Reagents that can be used as sources of Pd(O) are, for example, the combination of palladium diacetate-triphenylphosphine and a tertiary amine such as triethylamine or N-methylmorpholine, palladium dichloride diacetonitrilate and lithium chloride, and tetrakis-(triphenylphosphine)palladium (O). Other palladium compounds which can be reduced to Pd(O) are known and may be used in the process if otherwise compatible with the reactants.

The alcohol $R_2OH$ can be any straight or branched chain alkanol or phenyl substituted alkanol such as methanol, ethanol, n-propanol, iso-propanol, n-butanol, iso-butanol, n-hexanol, benzyl alcohol, 1-, or 2-phenylethanol, 3-phenylpropanol, 3-phenylbutanol, 4-phenylbutanol, 3-phenylhexanol, 6-phenylhexanol and like alkanols.

The process is preferably carried out under substantially anhydrous conditions with the reaction vessel,

solvent, starting material and reagents being substantially dry.

The process can be performed by adding the palladium (O) compound or the Pd(O) generating reagents to a solution of the triflate ester (2) in the inert solvent and the $R_2OH$ alkanol. The reaction mixture is then flushed with carbon monoxide until the reaction is complete. Alternatively, the solution of the triflate ester (2) in the solvent-alkanol mixture can be saturated with CO and the Pd(O) compound or Pd(O) source reagents added. The reaction mixture can be maintained in a CO atmosphere or periodically flushed with CO until the reaction is complete. The process can also be carried out under CO pressure e.g., from ca 15 psi to about 100 psi. Such pressures may be advantageous when the process is carried out on a large scale.

In an example of the process benzyl 7β-phenylacetylamino-3-trifluoromethylsulfonyloxy-1-carba(1-dethia)-3-cephem-4-carboxylate is dissolved in a 1:1 mixture of DMF and ethanol and palladium diacetate, triphenylphosphine in a 3-fold to 5-fold excess with respect to the Pd $(acetate)_2$ and an excess of triethylamine are added to the solution. Carbon monoxide is then bubbled through the reaction mixture until the reaction is complete.

The process can be followed by running thin layer chromatography on small aliquots of the reaction mixture from time to time. The 3-alkoxycarbonyl or 3-phenylalkoxycarbon-1-carba-3-cephem ester (1) is recovered from the reaction mixture by conventional isolation procedures. For example the reaction mixture is diluted with a water immiscible organic solvent such as ethyl acetate, the solution washed with dilute acid and bicarbonate and, after drying is evaporated to provide the reaction product in crude form. The product may be purified by chromatography e.g. over silica.

Examples of 3-alkoxycarbonyl- and 3-phenylalkoxycarbonyl-1-carba-3-cephem esters (1) are p-nitrobenzyl 7β-phenoxyacetylamino-3-ethoxycarbonyl-1-carba(1-dethia)-3-cephem-4-carboxylate, p-methoxybenzyl 7β-phenylacetylamino-3-methoxycarbonyl-1-carba(1-dethia)-3-cephem-4-carboxylate, diphenylmethyl 7β-t-butyloxycarbonylamino-3-isopropoxycarbonyl-1-carba(1-dethia)-3-cephem-4-carboxylate, t-butyl 7β-benzyloxycarbonylamino-3-(2-phenylethoxy)carbonyl-1-carba(1-dethia)-3-cephem-4-carboxylate, and benzyl 7β-formamido-3-benzyloxycarbonyl-1-carba(1-dethia)-3-cephem-4-carboxylate.

The 1-carba-3-triflate esters (2) used as the starting materials in the process are prepared by the method described by Evans et al. U.S. Patent No. 4,673,737. Although the mechanism by which the process of this invention proceeds is uncertain it may be described as the palladium catalyzed carbonylation of these triflate esters. The nature of the process suggests palladium insertion in the ester followed by displacement with CO.

In the above formula 1, when A is an acyl group R(CO)-, R is hydrogen; $C_1$-$C_6$ alkyl, $C_1$-$C_6$ alkyl substituted by cyano, carboxy, halogen, amino, $C_1$-$C_4$ alkoxy, $C_1$-$C_4$ alkylthio, or trifluoromethylthio; a phenyl or substituted phenyl group represented by the formula

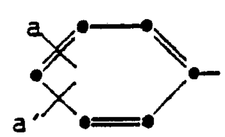

wherein a and a′ independently are hydrogen, halogen, hydroxy, $C_1$-$C_4$ alkoxy, $C_1$-$C_4$ alkanoyloxy, $C_1$-$C_4$ alkyl, $C_1$-$C_4$ alkylthio, amino, mono- or di($C_1$-$C_4$ alkyl)amino, $C_1$-$C_4$ alkanoylamino, $C_1$-$C_4$ alkylsulfonylamino, carboxy, carbamoyl, hydroxymethyl, aminomethyl, or carboxymethyl;

a group represented by the formula

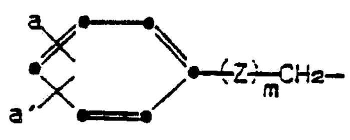

wherein a and a′ have the same meanings as defined above, Z is O or S, and m is 0 or 1;

a heteroarylmethyl group represented by the formula

$R^1$-$CH_2$-

wherein $R^1$ is thienyl, furyl, benzothienyl, benzofuryl, indolyl, triazolyl, tetrazolyl, oxazolyl, thiazolyl, oxadiazolyl, thiadiazolyl, and such heteroaryl groups substituted by amino, hydroxy, halogen, $C_1$-$C_4$ alkyl, $C_1$-$C_4$ alkoxy, $C_1$-$C_4$ alkylsulfonylamino;

a substituted methyl group represented by the formula

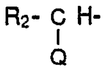

wherein $R^2$ is cyclohex-1,4-dienyl, or a phenyl group or substituted phenyl group represented by the formula

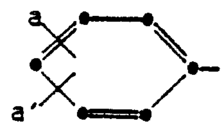

wherein a and a′ have the above-defined meanings, or $R^2$ is $R^1$ as defined above, and Q is hydroxy, $C_1$-$C_4$ alkanoyloxy, carboxy, sulfo, or amino;
or R is a keto group or an oximino-substituted group represented by the formulae

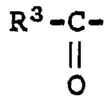

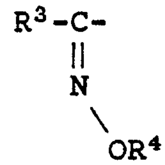

wherein $R^3$ is $R^1$ or $R^2$ as defined above and $R^4$ is hydrogen, $C_1$-$C_4$ alkyl, or a carboxy- substituted alkyl or cycloalkyl group represented by the formula

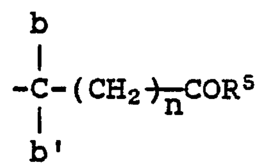

wherein b and b′ independently are hydrogen, or $C_1$-$C_3$ alkyl, and b and b′ when taken together with the carbon to which they are bonded form a 3- to 6-membered carbocyclic ring, and $R^5$ is hydroxy, $C_1$-$C_4$ alkoxy, amino, $C_1$-$C_4$ alkylamino, or di($C_1$-$C_4$ alkyl)amino.

In the above definition of the compounds represented by the formula 1, $C_1$-$C_6$ alkyl refers to the straight and branched chain alkyl groups such as methyl, ethyl, n-propyl, isopropyl, n-butyl, t-butyl, n-pentyl, n-hexyl, 3-methylpentyl, and like alkyl groups; $C_1$-$C_6$ alkyl substituted by cyano refers to cyanomethyl, cyanoethyl, 4-cyanobutyl, and the like; $C_1$-$C_6$ alkyl substituted by carboxy refers to such groups as carboxymethyl, 2-carboxyethyl, 2-carboxypropyl, 4-carboxybutyl, 5-carboxypentyl, and the like; $C_1$-$C_6$ alkyl substituted by halogen refers to chloromethyl, bromomethyl, 2-chloroethyl, 1-bromoethyl, 4-chlorobutyl, 4-bromopentyl, 6-chlorohexyl, 4-fluorobutyl, 3-fluoropropyl, fluoromethyl, and the like; $C_1$-$C_6$ alkyl substituted by amino refers to such groups as 2-aminoethyl, aminomethyl, 3-aminopropyl and 4-aminobutyl; $C_1$-$C_6$ alkyl substituted by $C_1$-$C_4$ alkoxy refers to methoxymethyl, 2-methoxyethyl, 2-ethoxyethyl, ethoxymethyl, 3-propoxypropyl, 3-ethoxybutyl, 4-t-butyloxybutyl, 3-methoxypentyl, 6-methoxyhexyl, and like group; $C_1$-$C_6$ alkyl substituted by $C_1$-$C_4$-alkylthio refers to such groups as for example methylthiomethyl, 2-methylthioethyl, 2-ethylthiopropyl, 4-methylthiobutyl, 5-ethylthiohexyl, 3-t-butylthiopropyl, and like groups; $C_1$-$C_6$ alkyl substituted by trifluoromethyl is exemplified by 2,2,2-trifluoroethyl, 3,3,3-trifluoropropyl, 4,4,4-trifluorobutyl, and the like; and $C_1$-$C_6$ alkyl substituted by trifluoromethylthio refers to, for example, trifluoromethylthiomethyl, 2-trifluoromethylthioethyl, 2-trifluoromethylthiopropyl, 4-trifluoromethylthiobutyl, 5-trifluoromethylthiohexyl, and like $C_1$-$C_6$ alkyl substituted groups.

When in the formula 1 R is a substituted phenyl group wherein the substituent(s) are represented by a and a′, examples of such groups are halophenyl such as 4-chlorophenyl, 3-bromophenyl, 2-fluorophenyl, 2,4-dichlorophenyl, and 3,5-dichlorophenyl; hydroxyphenyl such as 2-hydroxyphenyl, 3-hydroxyphenyl, 4-hydroxyphenyl, 2,4-dihydroxyphenyl, and 3,4-dihydroxyphenyl; alkoxyphenyl, such as 2,6-dimethoxyphenyl,

4-methoxyphenyl, 3-ethoxyphenyl, 3,4-dimethoxyphenyl, 4-t-butyloxyphenyl, 4-methoxy-3-ethoxyphenyl, and 4-n-propoxyphenyl; alkanoyloxyphenyl such as 2-acetoxyphenyl, 4-propionoxyphenyl, 4-formyloxyphenyl, 4-acetoxyphenyl, 3-butyryloxyphenyl, and 3-acetoxyphenyl; alkylphenyl such as 4-methylphenyl, 2-methylphenyl, 2,4-dimethyl phenyl, 3-t-butylphenyl, 4-ethylphenyl, 4-ethyl-3-methylphenyl, and 3,5-dimethylphenyl; alkylthiophenyl such as 4-methylthiophenyl, 3-n-butylthiophenyl, 2-ethylthiophenyl, 3,4-dimethylthiophenyl, and 3-n-propylthiophenyl; aminophenyl such as 2-aminophenyl, 4-aminophenyl, 3,5-diaminophenyl, and 3-aminophenyl; alkanoylamino such as 2-acetylamino, 4-acetylamino, 3-propionylamino, and 4-butyrylamino; alkylsulfonylamino such a 3-methylsulfonylamino, 4-methylsulfonylamino, 3,5-(dimethylsulfonylamino)phenyl, 4-n-butylsulfonylaminophenyl, and 3-ethylsulfonylaminophenyl; carboxyphenyl such as 2-, 3-, or 4-, carboxyphenyl, 3,4-dicarboxyphenyl, and 2,4-dicarboxyphenyl; carbamoylphenyl such as 2-carbamoylphenyl, 2,4-dicarbamoylphenyl, and 4-carbamoylphenyl; hydroxymethylphenyl such as 4-hydroxymethylphenyl and 2-hydroxymethylphenyl; aminomethylphenyl such as 2-aminomethylphenyl and 3-aminomethylphenyl; and carboxyphenyl such as 2-carboxymethylphenyl, 4-carboxymethylphenyl, and 3,4-dicarboxymethylphenyl; and the substituted phenyl groups bearing different substituents such as 4-chloro-3-methylphenyl, 4-fluoro-3-hydroxyphenyl, 3,5-dichloro-4-hydroxyphenyl, 4-hydroxy-3-chlorophenyl, 4-hydroxy-3-methylphenyl, 4-ethyl-3-hydroxyphenyl, 4-methoxy-3-hydroxyphenyl, 4-t-butyloxy-2-hydroxyphenyl, 4-acetylamino-3-methoxyphenyl, 3-amino-4-ethylphenyl, 2-aminomethyl-4-chlorophenyl, 2-hydroxymethyl-3-methoxyphenyl, 2-hydroxymethyl-4-fluorophenyl, 2-acetoxy-4-aminophenyl, 4-acetoxy-3-methoxyphenyl, 3-isopropylthio-4-chlorophenyl, 2-methylthio-4-hydroxy methylphenyl, 4-carboxy-3-hydroxyphenyl, 4-ethoxy-3-hydroxyphenyl, 4-methylsulfonylamino-2-carboxyphenyl, 4-amino-3-chlorophenyl, and 2-carboxymethyl-4-hydroxyphenyl.

Examples of RCO- groups of the formula 1 wherein R is a group represented by the formula

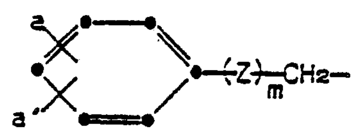

with m = 0 are: phenylacetyl, 4-hydroxyphenylacetyl, 4-chlorophenylacetyl, 3,4-dichlorophenylacetyl, 4-methoxyphenylacetyl, 3-ethoxyphenylacetyl, 2-aminomethylphenylacetyl, 3-carboxyphenylacetyl, 4-acetoxyphenylacetyl, 3-aminophenylacetyl, and 4-acetylaminophenylacetyl; and with m = 1 and Z = O, phenoxyacetyl, 4-chlorophenoxyacetyl, 4-fluorophenoxyacetyl, 3-aminophenoxyacetyl, 3-hydroxyphenoxyacetyl, 2-methoxyphenoxyacetyl, 2-methylthiophenoxyacetyl, 4-acetylaminophenoxyacetyl, 3,4-dimethylphenoxyacetyl, and 3-hydroxymethylphenoxyacetyl; and with m = 1 and Z = S, phenylthioacetyl, 4-chlorophenylthioacetyl, 3,4-dichlorophenylthioacetyl, 2-fluorophenylthioacetyl, 3-hydroxyphenylthioacetyl, and 4-ethoxyphenylthioacetyl.

Examples of $R^1$-$CH_2CO$-groups of the formula 1 wherein $R^1$ is a heteroaryl group are: 2-thienylacetyl, 3-thienylacetyl, 2-furylacetyl, 2-benzothienylacetyl, 2-benzofurylacetyl, indol-2-ylacetyl, 1H-tetrazol-1-ylacetyl, oxazol-2-ylacetyl, oxazol-4-ylacetyl, thiazol- 4-ylacetyl, 2-aminothiazol-4-ylacetyl, 1,3,4-oxadiazol-2-ylacetyl, 1,3,4-thiadiazol-2-ylacetyl, 5-ethyl-1,3,4-thiadiazol-2-ylacetyl, and like heteroaryl groups optionally substituted by amino, $C_1$-$C_4$ alkylsulfonylamino, hydroxy, halo, $C_1$-$C_4$ alkyl or $C_1$-$C_4$-alkoxy groups.

Examples of RCO- groups of the formula I compounds wherein R is a substituted methyl group represented by the formula $R^2$-CH(Q)- and Q is amino, carboxy, hydroxy, or sulfo, are 2-carboxy-2-phenylacetyl, 2-carboxy-2-(4-hydroxyphenyl)acetyl, 2-amino-2-phenylacetyl, 2-amino-2-(4-hydroxyphenyl)-acetyl, 2-amino-2-(3-chloro-4-hydroxyphenyl)acetyl, 2-amino-2-(cyclohex-1,4-dien-1-yl)acetyl, 2-hydroxy-2-phenylacetyl, 2-formyloxy-2-phenylacetyl, 2-sulfo-2-phenylacetyl, 2-sulfo-2-(4-methylphenyl)acetyl, and 2-acetoxy-2-(3-hydroxyphenyl)acetyl, 2-amino-2-(2-thienyl)acetyl, 2-amino-2-(3-benzothienyl)acetyl, 2-amino-2-(1H-tetrazol-1-yl)acetyl, 2-hydroxy-2-(1,3,4-thiadiazol-2-yl)acetyl, 2-amino-2-(2-aminothiazol-4-yl)acetyl, 2-carboxy-2-(2-thienyl)acetyl, 2-carboxy-2-(benzothien-2-yl)acetyl, and 2-hydroxy-2-(benzofur-2-yl)acetyl.

Examples of RCO acyl groups of the compounds represented by formula 1 when R is a keto group or an oximino-substituted group represented by the formulae

$$R^3-\underset{\underset{O}{\|}}{C}- \qquad\qquad R^3-\underset{\underset{N\diagdown OR^4}{\|}}{C}-$$

are the keto groups 2-oxo-2-phenylacetyl, 2-oxo-2-(2-thienyl)acetyl, 2-oxo-2-(2-aminothiazol-4-yl)acetyl; and oximino-substituted groups 2-phenyl-2-methoxyiminoacetyl, 2-(2-thienyl)-2-ethoxyiminoacetyl, 2-(2-furyl)-2-methoxyiminoacetyl, 2-(2-benzothienyl)-2-carboxymethoxyiminoacetyl, 2-(2-thienyl)-2-(2-carboxyethoxy)-iminoacetyl, 2-(2-amino-1,2,4-thiadiazol-4-yl)-2-methoxyiminoacetyl, 2-(2-aminothiazol-4-yl)-2-methoxyiminoacetyl, 2-(2-chlorothiazol-4-yl)-2-methoxyiminoacetyl, 2-(2-aminothiazol-4-yl)-2-(2-carboxyprop-2-yl)-oxyiminoacetyl, 2-(2-aminothiazol-4-yl)-2-(2-carbamoylprop-2-yl)oxyiminoacetyl, and 2-(5-amino-1,3,4-thiadiazol-2-yl)-2-methoxyiminoacetyl.

The starting material (2) desirably has any free amino or carboxy functions which may be present in the R(CO) group in protected form, e.g., protected with an $R_1$ protecting group or an amino-protecting group as defined below.

The carboxy-protecting group $R^1$ is a conventional carboxy-blocking group used in the $\beta$-lactam antibiotic art and serves the function of blocking the acidic carboxy group while reactions are carried out at other sites in the molecule. Such groups are used for the temporary protection or blocking of the carboxy group. Examples of such groups are t-butyl, haloalkyl groups, e.g. 2,2,2-trichloroethyl, 2-iodoethyl, benzyl, substituted benzyl, e.g. 4-nitrobenzyl, and 4-methoxybenzyl, diphenylmethyl, trialkylsilyl or mixed alkylarylsilyl groups, e.g. trimethylsilyl, triethylsilyl, dimethylphenylsilyl, $\beta$-trimethylsilylethyl, and $\beta$-methylsulfonylethyl.

Amino-protecting groups represented by A in the formulas 1 and 2 are the conventional protecting or blocking groups used in the $\beta$-lactam antibiotic art for the temporary protection of the amino group function while reactions at other sites in the molecule are carried out. Examples of suitable protecting groups are formyl, trichloroacetyl, tribromoacetyl, trityl, an alkyl, cycloalkyl, or aryloxycarbonyl group such as ethoxycarbonyl, t-butyloxycarbonyl, trichloroethoxycarbonyl, cyclopentyloxycarbonyl, benzyloxycarbonyl, p-nitrobenzyloxycarbonyl, and diphenylmethoxycarbonyl; allyloxycarbonyl, a bicyclooxycarbonyl group such as adamantyloxycarbonyl or bicycloheptyloxycarbonyl; or other conventional amino-protecting group. Preferred amino-protecting groups A are represented by the formula

$$R^0{}_1-O-\overset{\overset{O}{\|}}{C}-$$

wherein $R^0{}_1$ is $C_1$-$C_4$-alkyl, $C_3$-$C_7$ cycloalkyl, benzyl, nitrobenzyl, halobenzyl or methoxybenzyl. Preferred amino-protecting groups are benzyloxycarbonyl, p-nitrobenzyloxycarbonyl, and t-butyloxycarbonyl.

The 3-alkoxycarbonyl and 3-phenylakoxycarbonyl 1-carba esters (1) obtained in the process of this invention can be deesterified to provide the free $C_4$ carboxylic acid antibiotics. It is noted that the compounds provided by the process are diesters. The esterified $C_4$ carboxy group can be selectively deesterified by known procedures to provide the 3-alkoxycarbonyl-4-carboxylic acid antibacterial. For example, when $R_1$ is p-nitrobenzyl, treatment of the ester with zinc and acetic acid removes the ester providing the 3-alkoxycarbonyl or 3-phenylalkoxycarbonyl-3-cephem-4-carboxylic acid. The p-methoxybenzyl esters can be selectively removed with trifluoroacetic acid in anisole and the diphenylmethyl ester may be removed with formic acid. The 1-carba-3-cephem compounds formed in the process are described in European Patent Application No. 86310168.9, Publication No. 0232623. These 1-carba-3-cephem-4-carboxylic acids, the pharmaceutically acceptable salts and biologically labile esters thereof inhibit the growth of microorganisms pathogenic to man and animals.

In a preferred process of this invention $R_2$ is $C_1$-$C_4$ alkyl, especially methyl or ethyl, and A is an acyl group R(CO). Especially preferred acyl groups are phenoxyacetyl and phenylacetyl. Further preferred are triflates (2) and alkyloxycarbonyl esters (1) wherein A is an amino protecting group especially t-butyloxycarbonyl or benzyloxycarbonyl.

The following Examples are provided to further illustrate the invention and are not to be construed as limiting thereof.

## <u>Example 1</u>

Benzyl 7β-phenoxyacetylamino-3-methoxycarbonyl-1-carba(1-dethia)-3-cephem-4-carboxylate

To a solution of 68 mg (0.123 mmole) of benzyl 7β-phenoxyacetylamino-3-trifluoromethylsulfonyloxy-1-carba(1-dethia)-3-cephem-4-carboxylate in 1 ml of DMF and 2 ml of methyl alcohol were added palladium diacetate (5.6 mg, 0.025 mmole), triphenylphosphine (17 mg, 0.065 mmole) and triethylamine (36 µl, 0.258 mmole). The mixture was repeatedly evacuate and flushed with carbon monoxide until the reaction was complete as indicated by chromatograph (tlc). The reaction mixture was poured into 100 ml of ethyl acetate and the solution washed twice with 25 ml portions of 1N HCl and with brine, and was dried and evaporated to a dark oil. The oil was dissolved in methylene chloride and chromatographed over 10 g of silica eluting with ethyl acetate:hexane, 1:1, v:v. There were obtained 20 mg of the title compound. The product was dissolved in a mixture of 1 ml of methylene chloride and 5 ml of diethyl ether and, on adding a small volume of hexane (ca. 0.5 ml), 18 mg of the crystalline product was obtained.

90 MHz NMR ($CDCl_3$): δ 1.45, 2.0, 2.3 and 2.8 (m, 4H, $C_1H$ and $C_2H$), 3.5 (s, 3H, $OCH_3$), 3.9 (m, 1H, $C_6H$), 4.5 (s, 2H, $-OC\underline{H}_2-C$), 5.25 (s, 2H, $-C\underline{H}_2C_6H_5$), 5.4 (dd, J=5 and 7, 1H, $C_7H$), 6.8-7.5 (m, 10H, Ar).

IR (KBr): 1788 $cm^{-1}$ (β-lactam carbonyl)

Mass Spectrum (FD): 464 ($M^+$).

## Example 2

p-Nitrobenzyl 7β-phenoxyacetylamino-3-ethoxycarbonyl-1-carba(1-dethia)-3-cephem-4-carboxylate

p-Nitrobenzyl 7β-phenoxyacetylamino-3-trifluorosulfonyloxy-1-carba(1-dethia)-3-cephem-4-carboxylate (84 mg, 0.140 mmole) was reacted with the same reagents and under the same conditions as desribed by Example 1, except that ethyl alcohol was substituted for methyl alcohol. After 7 hours reaction time the mixture was diluted with ethyl acetate, the solution washed with 1N HCl, with dilute aqueous sodium bicarbonate and with brine and dried over sodium sulfate. The solution was concentrated by evaporation and the concentrate chromatographed over 20 g of silica (ethyl acetate:hexane, 1:1, v:v) yielding 19 mg (26% yield) of the title compound.

90 MHz NMR ($CDCl_3$): δ 1.2 (t, J=7, 3H, $CCH_3$), 1.4, 2.0, 2.3 and 2.8 (m, 4H, $C_1H$ and $C_2H$), 3.9 (m, 1H, $C_6H$), 4.1 (q, J=7, 2H, $-OC\underline{H}_2CH_3$), 4.5 (s, 2H, $C_6H_5OC\underline{H}_2CO$), 5.3 (m, 3H, $ArC\underline{H}_2$ and $C_7H$), 6.7-7.4 (m, $C_6H$, $C_6\underline{H}_5OC$ and $N\underline{H}$), 7.5 and 8.1 (d, J=9, 4H, $C_6\underline{H}_4NO_2$).

Mass Spectrum (FD): 523 ($M^+$).

## Example 3

p-Nitrobenzyl 7β-phenoxyacetylamino-3-(m-propoxycarbonyl)-1-carba(1-dethia)-3-cephem-4-carboxylate

p-Nitrobenzyl 7β-phenoxyacetylamino-3-trifluoromethylsulfonyloxy-1-carba(1-dethia)-3-cephem-4-carboxylate (15 g, 25 mmole), palladium diacetate (0.86 g, 3.83 mmole), triphenylphosphine (2.4 g, 9.19 mmole) were dissolved in 220 ml of n-propyl alcohol and 220 ml of DMF. To the solution were added with stirring 3.6 ml of triethylamine and 2.8 ml of N-methylmorpholine and carbon monoxide was bubbled through the solution for about 8 minutes. The reaction mixture was then allowed to stir for about 16 hours under a positive pressure of carbon monoxide (balloon pressure). The solution was diluted with 1.5 l of ethyl acetate and washed with 1N HCl, dilute aqueous sodium bicarbonate, again with 1N HCl, and with brine and was dried over sodium sulfate. After the solution was concentrated by evaporation, the concentrate was chromatographed over 500 g of silica (ethyl acetate:hexane, 6:8, to ethyl acetate:hexane, 8:6, v:v) to yield 3.8 g (28% yield) of the title compound.

The 3-(n-propoxycarbonyl)-1-carba-3-cephem ester was dissolved in a mixture of 30 ml of acetic acid and 100 ml of methylene chloride and 2.3 g of zinc were added. The solution was warmed with stirring for 1 hour and when thin layer chromatography showed the presence of some starting material, another 10 ml of

acetic acid and 1 g of zinc were added. After another hour, another 10 ml of acetic acid and 1 g of zinc were added to complete the deesterification as shown by thin layer chromatography. The reaction mixture was concentrated by evaporation, the concentrate dissolved in ethyl acetate and concentrated again by evaporation. The concentrate was diluted with ethyl acetate and the solution washed with 1N HCl and then three times with 25 ml portions of saturated aqueous sodium bicarbonate. The organic layer was discarded and methylene chloride was added to the aqueous layer. The aqueous layer was acidified and the organic layer separated. After washing with brine and drying, the organic layer was evaporated under vacuum yielding 1.7 g (60% yield) of 7β-phenoxyacetylamino-3-(n-propoxycarbonyl)-1-carba(1-dethia)-3-cephem-4-carboxylic acid.

The acid product was reesterified to the diphenylmethyl ester as follows: The acid, 1.8 g, was dissolved in 50 ml of acetonitrile and 0.96 g of diphenyldiazomethane was added slowly. Gas evolution occurred and the solution turned purple. After stirring at room temperature for 1.5 hour, acetic acid was added until the purple color was dissipated leaving a yellow solution. The solution was concentrated under vacuum and the concentrate chromatographed over about 250 g of silica using initially hexane:ethyl acetate, 2:1 and changing to hexane:ethyl acetate, 60:40, v:v. There were obtained 2.2 g (87%) of the ester, diphenylmethyl 7β-phenoxyacetylamino-3-(n-propoxycarbonyl)-1-carba(1-dethia)-3-cephem-4-carboxylate.
300 MHz NMR ($CDCl_3$): δ 0.7 (t, J=7, 3H, $OCCC\underline{H}_3$), 1.3 (m, 2H, $OC\underline{H}_2CH_3$), 1.3, 1.95, 2.25 and 2.8 (m, 4H, $C_1H$ and $C_2H$), 3.6 and 3.8 (m, 2H, $-OC\underline{H}_2\overline{CH}_2CH_3$), 3.9 (m, 1H, $C_6H$), 4.45 (s, 1H, $C_6H_5OC\underline{H}_2C$), 5.4 (dd, J=5 and 8, 1H, $C_7H$), 6.8-7.4 (m, 17H, ArH, NH and $-C\underline{H}(C_6H_5)_2$).
Mass Spectrum (FD): 586 ($M^+$).

## Example 4

p-Nitrobenzyl 7β-phenoxyacetylamino)-3-(iso-propoxycarbonyl)-1-carba(1-dethia)-3-cephem-4-carboxylate

p-Nitrobenzyl 7β-phenoxyacetylamino-3-trifluoromethylsulfonyloxy-1-carba(1-dethia)-3-cephem-4-carboxylate, 3 g, was reacted with the reagents and under the conditions employed in Example 3, except that iso-propyl alcohol was substituted for the n-propyl alcohol of Example 3 to provide after chromatography 330 mg (12%) of the title compound as an oil.
90 MHz NMR ($CDCl_3$): δ 1.3 (d, J=7, 6H, $(C\underline{H}_3)_2CH-$), 1.5, 2.0 2.3 and 2.8 (m, 4H, $C_1H$ and $C_2H$), 3.9 (m, 1H, $C_6H$), 4.5 (s, 2H, $C_6H_5OC\underline{H}_2CO$), 5.0 (m, 1H, $(CH_3)_2C\underline{H}O-$), 5.35 (m, 3H, $C_7H$ and $C\underline{H}_2Ar$), 6.8-7.4 (m, 6H, $-OC_6\underline{H}_5$ and $N\underline{H}$), 7.55 and 8.15 (d, J=8, 4H, $C_6\underline{H}_4NO_2$).

## Example 5

p-Nitrobenzyl 7β-phenoxyacetylamino-3-(iso-butoxycarbonyl)-1-carba(1-dethia)-3-cephem-4-carboxylate

The title compound was obtained by using the reagents and conditions described in Examples 3 and 4, except that iso-butyl alcohol was used instead of n- or iso-propyl alcohol. With 15.3 g of the triflate ester there were obtained after silica chromatography 3.3 g (23%) of the title compound.

The 3-iso-butoxycarbonyl p-nitrobenzyl ester product was deesterified with zinc and acetic acid to yield 1.3 g of the free acid.

The free acid compound, 1.3 g, was esterified in acetonitrile with diphenyldiazomethane to yield 1.62 g of diphenyl 7β-phenoxyacetylamino-3-(iso-butoxycarbonyl)-1-carba(1-dethia)-3-cephem-4-carboxylate.
300 MHz NMR ($CDCl_3$): δ 0.8 (m, 6H, $-CH(C\underline{H}_3)_2$), 1.4, 2.0, 2.3 and 2.85 (m, 4H, $C_1H$ and $C_2H$), 1.6 (m, 1H, $-CH_2C\underline{H}(CH_3)_2$), 3.5 and 3.75 (dd, J=6 and 10, 2H, $-OC\underline{H}_2CH(CH_3)_2$), 3.95 (m, 1H, $C_6H$), 4.55 (s, 2H, $C_6H_5OC\underline{H}_2-CO$), 5.45 (dd, J=5 and 8, 1H, $C_7H$, 6.9-7.5 (m, 17H, Ar$\underline{H}$, N$\underline{H}$ and $-C\underline{H}(C_6H_5)_2$).

Example 6

p-Nitrobenzyl 7β-phenoxyacetylamino)-3-methoxycarbonyl-1-carba(1-dethia)-3-cephem-4-carboxylate

A dry solution of 1.0 g (1.67 mmole) of p-nitrobenzyl 7β-phenoxyacetylamino-3-trifluoromethylsulfonyloxy-1-carba(1-dethia)-3-cephem-4-carboxylate and 212 mg (5.0 mmole) of lithium chloride in a mixture of 15 ml of DMF and 15 ml of methyl alcohol was flushed with carbon monoxide and 0.46 ml (3.34 mmole) of triethylamine was added followed by 143 mg (0.55 mmole) of palladium dichloride di-acetonitrilate. A black precipitate formed and the reaction mixture was stirred for 2 hours at room temperature. The mixture was diluted with 250 ml of ethyl acetate and the solution washed sequentially with 1N HCl, saturated aqueous sodium bicarbonate, 1N HCl and brine. The washed solution was dried, concentrated under vacuum and the concentrate passed through a 2-inch column packed with silica gel using ethyl acetate:hexane, 60:40, v:v. There were obtained 113 mg of starting material and 492 mg of the title compound (65% yield based on recovered starting material).

## Claims

1. A process for preparing a compound of formula 1

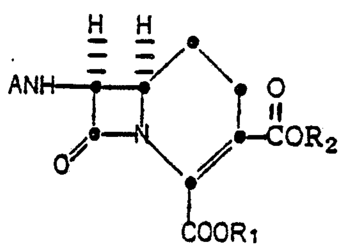

1

wherein A is an amino-protecting group or an acyl group R(CO); $R_1$ is a carboxy-protecting group; and $R_2$ is $C_1$-$C_6$ alkyl or $C_1$-$C_6$ alkyl substituted by phenyl; which comprises reacting a 1-carba(1-dethia)-3-cephem-3-triflate ester represented by the formula

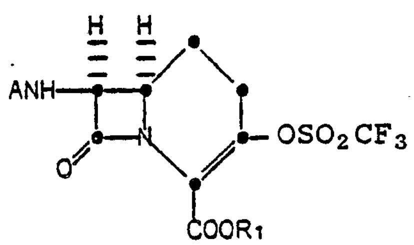

wherein A and $R_1$ have the same meanings as defined above, with carbon monoxide and a $C_1$-$C_6$ alkanol or a phenyl-substituted $C_1$-$C_6$ alkanol in the presence of palladium (O);
wherein R is hydrogen; $C_1$-$C_6$ alkyl, $C_1$-$C_6$ alkyl substituted by cyano, carboxy, halogen, amino, $C_1$-$C_4$ alkoxy, $C_1$-$C_4$ alkylthio, or trifluoromethylthio; a phenyl or substituted phenyl group represented by the formula

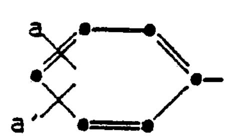

wherein a and a′ independently are hydrogen, halogen, hydroxy, $C_1$-$C_4$ alkoxy, $C_1$-$C_4$ al-

kanoyloxy, $C_1$-$C_4$ alkyl, $C_1$-$C_4$ alkylthio, amino, mono- or di($C_1$-$C_4$ alkyl)amino, $C_1$-$C_4$ alkanoylamino, $C_1$-$C_4$ alkylsulfonylamino, carboxy, carbamoyl, hydroxymethyl, aminomethyl, or carboxymethyl;

a group represented by the formula

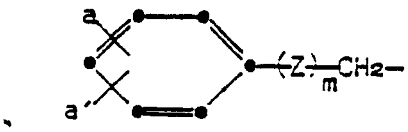

wherein a and a′ have the same meanings as defined above, Z is O or S, and m is 0 or 1;

a heteroarylmethyl group represented by the formula

$R^1$-$CH_2$-

wherein $R^1$ is thienyl, furyl, benzothienyl, benzofuryl, indolyl, triazolyl, tetrazolyl, oxazolyl, thiazolyl, oxadiazolyl, thiadiazolyl, and such heteroaryl groups substituted by amino, hydroxy, halogen, $C_1$-$C_4$ alkyl, $C_1$-$C_4$ alkoxy, $C_1$-$C_4$ alkylsulfonylamino;

a substituted methyl group represented by the formula

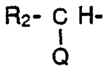

wherein $R^2$ is cyclohex-1,4-dienyl, or a phenyl group or substituted phenyl group represented by the formula

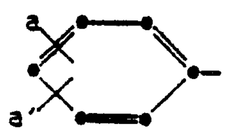

wherein a and a′ have the above defined meanings, or $R^2$ is $R^1$ as defined above, and Q is hydroxy, $C_1$-$C_4$ alkanoyloxy, carboxy, sulfo, or amino;

or R is a keto group or an oximino-substituted group represented by the formulae

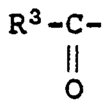

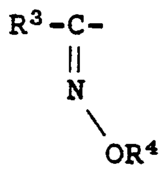

wherein $R^3$ is $R^1$ or $R^2$ as defined above and $R^4$ is hydrogen, $C_1$-$C_4$ alkyl, or a carboxy-substituted alkyl or cycloalkyl group represented by the formula

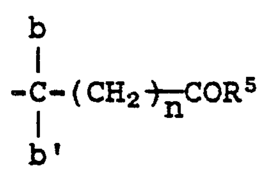

wherein b and b′ independently are hydrogen, or $C_1$-$C_3$ alkyl, and b and b′ when taken together with the carbon to which they are bonded form a 3- to 6-membered carbocyclic ring, and $R_5$ is hydroxy, $C_1$-$C_4$ alkoxy, amino, $C_1$-$C_4$ alkylamino, or di($C_1$-$C_4$ alkyl)amino.

2. A process of claim 1 wherein $R_2$ is $C_1$-$C_4$ alkyl.

3. A process of claim 1 or 2 wherein A is an acyl group R(CO).

4. A process of claim l or 2 wherein A is an amino-protecting group.

5. A process of any one of claims 1 to 4 wherein R is benzyl or phenoxymethyl and $R_2$ is methyl, ethyl, n-propyl or iso-propyl.

6. A process of any one of claims 1 to 5 wherein the Pd(O) is provided with palladium diacetate, triphenylphosphine and a tertiary alkyl amine.

7. A process of any one of claims 1 to 5 wherein the Pd(O) is provided with $PdCl_2 \cdot 2$ acetonitrile, lithium chloride and tertiary amine.